# EUROPEAN PATENT SPECIFICATION

(11) **EP 1 931 317 B1**
(45) Date of publication and mention of the grant of the patent: **24.12.2008**
(21) Application number: 06778242.5
(22) Date of filing: 15.08.2006
(51) Int. Cl.: A61K 9/36, A61K 38/46

(54) **PANCREATIN MICROPELLETS SUITABLE FOR ENTERIC COATING**
PANKREATIN-MIKROPELLETS GEEIGNET FÜR MAGENSAFTRESISTENTE ÜBERZÜGE
MICROGRANULES DE PANCREATINE ADAPTES A UN ENROBAGE ENTERIQUE

(30) Priority: 15.08.2005 EP 05107474; 15.08.2005 US 708526 P
(43) Date of publication of application: 18.06.2008
(73) Proprietor: Solvay Pharmaceuticals GmbH, 30173 Hannover (DE)
(72) Inventor: SHLIEOUT, George, 31319 Sehnde (DE); KOELLN, Claus-Juergen, 31535 Neustadt (DE); SCZESNY, Frithjof, 30173 Hannover (DE); ONKEN, Jens, 30890 Barsinghausen (DE); RUESING, Guido, 31535 Neustadt (DE)
(74) Representative: Bauriegel, Lutz
(86) International application number: PCT/EP2006/065313
(87) International publication number: WO 2007/020260

(56) References cited:
- DE-A1- 19 907 764
- US-A- 5 378 462
- US-A1- 2002 061 302
- US-A1- 2004 101 562

## Description

A process for the manufacture and use of a medicament containing pancreatin is described herein. More specifically, processes for manufacturing pancreatin micropellet cores which are substantially free of synthetic oils and pancreatin micropellet cores obtainable according to that process are described. Also described herein are pancreatin micropellets which are enteric-coated pancreatin micropellet cores.

Pancreatin microspheres are the treatment of choice for diseases or disorders caused by digestive enzyme deficiency in mammals such as humans. This is due to the fact that high-performance pancreatin microsphere products like Creon^{™} provide a therapeutically effective load of active enzymes while at the same time providing properly sized microspheres capable of targeting the optimal location in the digestive tract where digestive enzyme activity will be needed, in particular the upper intestine.

Recently, health authorities have initiated a reassessment of the compatibility of certain pharmaceutical excipients which had previously been used in the formulation of i.a. pancreatin-containing products. As a result, some health authorities have provided advice concerning specific pharmaceutical excipients (see e.g. US Code of Federal Regulations, 21 CFR §201.302), such as mineral oil. It is recommended today that mineral oil not be provided indiscriminately to pregnant women and/or to infants. Thus, there is a need provide patients with a pancreatin micropellet product in compliance with the current advice of the health authorities and which does not include synthetic oils such as mineral oil.

Synthetic oils like paraffins, e.g. liquid paraffins (mineral oils), in particular highly liquid paraffin (light mineral oil) have previously been understood to be a necessary excipient for manufacturing pancreatin micropellet products by extrusion and subsequent spheronisation of the extrudates. One example is described in document EP 0 583 726 (U.S. Pat. No. 5,378,462), which discloses pancreatin micropellets and their preparation with polyethylene glycol 4000, paraffin and a lower alcohol, by extrusion and subsequent spheronisation.

United States Pat. App. No. 2004/0101562 (Maio) discloses microspheres of pancreatic enzymes with high stability and a production method thereof. A solid mixture, including one or more pancreatic enzymes, one or more hydrophilic low-melting polymers and other excipients, is heated at a temperature equal or higher than the melting temperature of said hydrophilic low-melting polymer while stirring. However, Maio emphasizes that a fundamental feature of the process described therein is the total absence of any solvents, either water or other organic solvents.

In United States Pat. App. No. 200210061302 a method for the treatment of diabetes by administering a physiologically acceptable enzyme mixture having lipolytic, proteolytic and amylolytic activity to a patient in need thereof is described.

US patent application No. 2004/0213847 relates to delayed pharmaceutical compositions containing proton pump inhibitors.

US patent No. 4,786,505 teaches pharmaceutical preparations for oral use.

Further pharmaceutical preparations which may comprise pancreatin and an enteric coating are e.g. known from documents DE 19907764; EP 0 021 129 (U.S. Pat. No. 4,280,971); EP 0 035 780; U.S. Pat. No, 5,225,202; U.S. Pat. No. 5,750,148; U.S. 6,224,910; U.S. Pat. App. No. 2002/0146451 or WO 02/40045.

Accordingly, one embodiment disclosed herein is a process for making and using pancreatin micropellets cores which are substantially free of synthetic oils. Another embodiment provides pancreatin micropellets substantially free of synthetic oils which are enteric-coated pancreatin micropellet cores.

Another embodiment provides a method of treating various medical conditions such as pancreatic exocrine insufficiency, pancreatitis, cystic fibrosis, diabetes type I and diabetes type II by using the pancreatin micropellet cores and/or pancreatin micropellets obtained by the processes described herein.

Another embodiment provides a pharmaceutical composition in an oral dosage form containing a pharmacologically effective amount of pancreatin wherein the pancreatin is in the form of pancreatin micropellet cores and/or pancreatin micropellets manufactured according to the processes described herein. The pancreatin micropellet cores, the pancreatin micropellets and/or their pharmaceutical compositions can further be incorporated in at least one outer package selected from capsules, sachets, blisters or bottles.

Pancreatin is a mixture of different physiologically active endogenous ingredients which are derived from mammalian pancreas glands and comprised of several different digestive enzymes such as lipases, amylases and proteases. Mammalian pancreatic lipase is a valuable digestive enzyme supplement for the treatment of various medical conditions such as pancreatic exocrine insufficiency. However, pancreatic proteases and amylases also contribute to the therapeutic value of pancreatin. Pancreatin for pharmaceutical use is typically of bovine or porcine origin with porcine pancreatin being preferred.

It has now been surprisingly found that pancreatin micropellet cores which are suitable for enteric coating, are high in enzymatic activity and are substantially free of synthetic oils like paraffins, e.g. highly liquid paraffin, can be produced by the processes described herein. It has further been found that the manufacturing process described herein is an improvement when compared to known processes which use mineral oil or known processes which would e.g. need more process steps to produce pancreatin micropellet cores.

In particular, pancreatin micropellet cores can be produced by the process described herein which comprise 10% to 95% by weight of pancreatin, 5% to 90% by weight of at least one pharmaceutically acceptable binding agent and 0% to 10% by weight of at least one pharmaceutically acceptable excipient. More specifically, pancreatin micropellet cores can be produced by the process described herein which comprise 70% to 90% by weight of pancreatin, 10% to 30% by weight of at least one pharmaceutically acceptable binding agent and 0% to 5% by weight of at least one pharmaceutically acceptable excipient. In one embodiment, pancreatin micropellet cores can be produced which comprise 70% to 90% by weight pancreatin, and 10% to 30% by weight of at least one pharmaceutically acceptable binding agent, it being understood that the constituents of all aforementioned compositions add to 100 % by weight in each case.

For the purposes of the present disclosure, the prefix "micro" used to describe a micropellet or a microsphere, means that the diameter or each of the individual dimensions (length, height, width) is equal to or less than 5 mm. Producing pancreatin micropellet cores which are approximately spherical and have a diameter of 0.5 to 2.0 mm is preferred.

The term "synthetic oils" means unsaponifiable hydrocarbons or mixtures of hydrocarbons and comprises e.g. liquid and solid paraffins, in particular liquid paraffins (mineral oils), more particularly highly liquid paraffin (light mineral oil).

The phrase "substantially free of synthetic oils" means that the manufacturing processes described herein and used to make the pancreatin micropellet cores and/or pancreatin micropellets do not utilize one or more synthetic oils as an excipient although synthetic oils may be present as pharmaceutically acceptable trace contaminants in the binding agent(s), enteric coating constituents, the enzyme-friendly organic solvents and/or excipients which are used to manufacture the pancreatin micropellet cores and/or pancreatin micro pellets described herein.

One embodiment described herein is a process for the manufacture of pancreatin micro pellet cores, comprising the steps of:
a. preparing an extrudable mixture comprising:
   i. 10% to 95% pancreatin;
   ii. 5% to 90% of at least one pharmaceutically acceptable binding agent;
   iii. 0% to 10% of at least one pharmaceutically acceptable excipient; and
   iv. one or more enzyme-friendly organic solvents in an amount sufficient to form an extrudable mixture;
   wherein the percentages of components are weight to weight of the pancreatin micropellet cores and the constituents i.), ii.) and iii.) (if present) add to 100 % by weight;
b. creating pancreatin micropellets cores from the extrudable mixture;
c. forming the pancreatin micropellet cores into approximately spherical or approximately ellipsoidal shape in the presence of additional enzyme-friendly organic solvent; and
d. removing the one or more enzyme-friendly organic solvents from the pancreatin micropellets cores such that the pancreatin micropellet cores are substantially free of the one or more enzyme-friendly organic solvents;
wherein the pancreatin micropellet cores are substantially free of synthetic oils.

Examples of pharmaceutically acceptable binding agents used in process step a.) include polyethylene glycol 1500, polyethylene glycol 2000, polyethylene glycol 3000, polyethylene glycol 4000, polyethylene glycol 6000, polyethylene glycol 8000, polyethylene glycol 10000, hydroxypropyl methylcellulose, polyoxyethylen, copolymers of polyoxyethylen-polyoxypropylen and mixtures of said organic polymers. The foregoing list of pharmaceutically acceptable binding agents is not meant to be exhaustive, but merely illustrative as a person of ordinary skill in the art would understand that many other pharmaceutically acceptable binding agents or combinations of binding agents could also be used. Polyethylene glycol 4000 is the preferred pharmaceutically acceptable binding agent. For the purposes of the present disclosure, synthetic oils are not to be regarded as suitable pharmaceutically acceptable binding agents.

Examples of suitable pharmaceutically acceptable excipients include gliding agents like magnesium stearate or calcium stearate, stearic acid, talcum and/or starch; fillers like calcium phosphate, corn starch, dextrans, dextrin, hydrated silicon dioxide, microcrystalline cellulose, kaolin, lactose, mannitol, polyvinyl pyrrolidone, precipitated calcium carbonate, sorbitol and/or talcum; disintegrating agents like Aerosil^{™} (silicic acid), alginic acid, amylose, calcium alginate, calcium carbonate, formaldehyde gelatin, pectic carbonate, sago starch, sodium bicarbonate and/or starch; and/or moisturizers like glycerol and/or starch. The foregoing list of pharmaceutically acceptable excipients is not meant to be exhaustive, but merely illustrative as a person or ordinary skill in the art would understand that many other pharmaceutically acceptable excipients or combination of excipients could also be used. For the purposes of the present disclosure, synthetic oils are not to be regarded as suitable pharmaceutically acceptable excipients. In one embodiment, the pancreatin micropellet cores contain no pharmaceutically acceptable excipients but can optionally contain a higher load or dose of pancreatin.

Process variations wherein the pharmaceutically acceptable excipients are present in an amount of 0% are preferred.

Enzyme-friendly organic solvents facilitate mixing and other processing procedures and may afterwards be removed, for example, by drying. Typically, after removal of the enzyme-friendly organic solvents a certain amount of solvent remains in the pancreatin micropellet cores. The remaining solvent in the micropellet cores can comprise enzyme-friendly organic solvents, water, or a mixture of enzyme-friendly organic solvents with water. If water is present as a solvent, this will typically have been present in the pancreatin which was used as the starting material. The amount of solvent present in the pancreatin micropellet cores after removal of the enzyme-friendly organic solvents is typically less than 5 % and normally less than 3 % by weight of the pancreatin micropellet core.

Examples of suitable enzyme-friendly organic solvents are acetone, chloroform, dichloromethane or straight-chained or branched C₁₋₄-alcohols, particularly methanol, ethanol, 1-propanol, 2-propanol, 2-butanol, tert.-butanol or mixtures of said solvents. 2-propanol is the preferred enzyme-friendly organic solvent. For the purposes of the present disclosure, synthetic oils are not to be regarded as suitable enzyme-friendly organic solvents. The enzyme-friendly organic solvent is typically used in an amount of 15% to 35% by weight, preferably of 20% to 30% by weight, relative to the amount of pancreatin used. The foregoing list of suitable enzyme-friendly organic solvents is not meant to be exhaustive, but merely illustrative as a person of ordinary skill in the art would understand that many other enzyme-friendly organic solvents or combinations of solvents could also be used.

The amounts of pancreatin, pharmaceutically acceptable binding agent(s), pharmaceutically acceptable excipient(s) and/or enzyme-friendly organic solvent may be varied by those skilled in the art to arrive at the pancreatin micropellet cores having the preferred composition and characteristics as indicated herein.

The term substantially free of enzyme-friendly organic solvents" means that the quantity of enzyme-friendly organic solvents present in the pancreatin micropellet cores would be less than 5% by weight of the pancreatin micropellet core.

Removal of the one or more enzyme-friendly organic solvents from the pancreatin micropellet cores in process step d.) means that said pancreatin micropellet cores are subject to conditions whereby the micropellet cores become substantially free from enzyme-friendly organic solvents. Removal of the enzyme-friendly organic solvents can be by any method known to those of ordinary skill in the art. The preferred method is by drying. Drying can e.g. be performed at a temperature from 25 °C to 75 °C, preferably from 30 °C to 55 °C and for o period of e.g. 6 hours to 18 hours. Additionally, removal of the one or more enzyme-friendly organic solvents would also typically result in the pancreatin micropellet cores containing an amount of water which is less than 5% and typically less than 3% by weight of the pancreatin micropellet core.

In a preferred embodiment of the disclosed process for the manufacture of pancreatin micropellet cores the pancreatin micropellet cores are created in process step b.) by extrusion. Remarkably, an extrudable mixture is obtained even though the mixture is substantially free of synthetic oils. In process step b.), if the creating of the micropellet cores from the extrudable mixture is accomplished by means of extrusion, then the temperature preferably does not exceed 70°C during extrusion, more preferably the temperature does not exceed 50 °C. Also, in the event of extrusion, piercing dies are preferably used which have a hole diameter of 0.5 mm to 2.0 mm, preferably of 0.7 mm to 1.5 mm, e.g. 0.8 mm. If the extrudable mixture is extruded, then the extrudate fragments are brought to a suitable length for the forming. This can be done e.g. by means of a cutting device arranged downstream to the extruding press in a manner known to the a person of ordinary skill in the art. The forming in process step c.) can be carried out e.g. in a customary rounding apparatus. In the rounding apparatus, the extrudate fragments are then formed into an approximately spherical or approximately ellipsoidal shape in the presence of additional enzyme-friendly organic solvent which may be the same or different than the enzyme-friendly organic solvent used in process step a.).

When prepared as described herein (substantially free of synthetic oils), processing of the extrudate fragments in the rounding apparatus is improved relative to other known processes. For example, a lower amount of enzyme-friendly organic solvent needs to be added when forming the pancreatin micropellet cores into an approximately spherical or approximately ellipsoidal shape and fewer of the extrudate fragments stick to parts of the rounding apparatus when the process is practiced with an extruder and rounding apparatus.

A further embodiment comprises pancreatin micro pellets which are enteric-coated pancreatin micropellet cores. For enteric coating, any enteric coating can be used which is suitable for delivery of the pancreatin micropellet cores to the upper intestine and compatible with the pancreatin micropellet cores. Examples are enteric coatings known from U.S. Pat. No. 5,378,462 or commercially available enteric coatings like Eudragit™ polymers. Preferred enteric coatings are ones that would not require the presence of synthetic oils.

It has been found that the pancreatin micropellet cores and the pancreatin micropellets produced according to the processes disclosed herein and not using synthetic oils unexpectedly show essentially the same properties as pancreatin micropellet cores and pancreatin micro pellets produced according to known processes using mineral oil such as the processes disclosed in U.S. Pat. No. 5,378,462. In particular, the pancreatin micropellet cores and the pancreatin micropellets produced without using synthetic oils have a similar particle size distribution, bulk density and are obtained in similar yields as the pancreatin micropellet cores and pancreatin micropellets produced according to processes which use synthetic oils. Further, the pancreatin micropellets cores produced without using synthetic oils, when compared to similar pancreatin micropellets using synthetic oils, show similar appearances in their surface structures and a similar performance when coated with an enteric coating to give pancreatin micropellets.

In another embodiment, the enteric coating on the pancreatin micropellets cores comprises:
i) at least one film-forming agent;
ii) at least one plasticizer; and
iii) optionally at least one anti-sticking agent.

In one embodiment the enteric coating comprises between 20% and 30% by weight, more preferably between 22% and 26% by weight, yet more preferably between 22.5 % and 25 % by weight of the total composition of the pancreatin micropellet.

Film-forming agent(s), plasticizer(s) and anti-sticking agent(s) (when present) as used for preparing the enteric coating are hereinafter commonly referred to as "non-solvent coating constituents".

Suitable film-forming agents include agar, Carbopol™ (carbomer) polymers (i.e. high molecular weight, crosslinked, acrylic acid-based polymers), carboxymethyl cellulose, carboxymethylethyl cellulose, carrageen, cellulose acetate phthalate, cellulose acetate succinate, cellulose acetate trimelliate, chitin, corn protein extract, ethyl cellulose, gum arabic, hydroxypropyl cellulose, hydroxypropylmethyl acetate succinate, hydroxypropyl methylcellulose acetate succinate, hydroxypropyl methylcellulose phthalate, methacrylic acid-ethyl methacrylate-copolymer, methyl cellulose, pectin, polyvinyl acetate phthalate, polivinyl alcohol, shellac, sodium alginate, starch acetate phthalate and/or styrene/maleic acid copolymer or mixtures of said film-forming polymers. Cellulose acetate phthalate, hydroxypropyl methylcellulose acetate succinate and/or methacrylic acid-ethyl methacrylate-copolymer are the preferred film-forming agents. Most preferred is hydroxypropyl methylcellulose phthalate, e.g. HP 55 or HPMCP HP-50. Synthetic oils are not to be regarded as preferred film-forming agents. The foregoing list of film-forming agents is not meant to be exhaustive but merely illustrative, as a person or ordinary skill in the art would understand that many other film-forming agents or combination of film-forming agents could also be used.

The plasticizer(s) may generally be present in an amount greater than 1.5 %, and typically in an amount of 2% to 20% by weight, relative to the film-forming agent. The plasticizer may contain saturated linear monohydric alcohols having 12 to 30 carbon atoms. More specifically, acceptable plasticizers include lauryl alcohol, tridecyl alcohol, myristyl alcohol, pentadecyl alcohol, cetyl alcohol, heptadecyl alcohol, stearyl alcohol, nonadecyl alcohol, arachic alcohol, behenyl alcohol, carnaubyl alcohol, ceryl alcohol, corianyl alcohol, melissyl alcohol, acetyl tributyl citrate, dibutyl sebacate, fatty acid esters of glycerol, glycerol, polyethylene glycol, propyleneglycol, sorbitan fatty acids, triacetin, triethyl citrate and mixtures of said plasticizers. Preferred plasticizers are cetyl alcohol, stearyl alcohol, triethyl citrate and mixtures thereof. Most preferred plasticizers are selected from the group consisting of triethyl citrate, cetyl alcohol and mixtures of triethyl citrate and cetyl alcohol. When cetyl alcohol is used as a single plasticizer, it may be present in an amount of greater than 1.5 %, typically in an amount of 2% to 15%, preferably 2% to 10%, by weight relative to the film-forming agent. When triethyl citrate is used as a single plasticizer, it may be present in an amount of 5% to 20%, preferably 12% to 15%, by weight relative to the film-forming agent. Synthetic oils are not to be regarded as preferred plasticizers. The foregoing list of plasticizers is not meant to be exhaustive but merely illustrative, as a person or ordinary skill in the art would understand that many other plasticizers or combination of plasticizers could also be used.

In one embodiment the plasticizer is comprised of cetyl alcohol and triethyl citrate which are collectively present in an amount of greater than 3%, typically in an amount of 4% to 20%, in particular between 6% and 15%, more particularly between 7% and 10%, by weight in relation to the film-forming agent. The weight to weight ratio of cetyl alcohol to triethyl citrate in said mixture of cetyl alcohol and triethyl citrate may be from 0.05:1 to 1:1, for example 0.1:1, 0.2:1, 0.3:1, 0.4:1, 0.5:1, 0.6:1, 0,7:1, 0.8:1 or 0.9:1. In particular, the weight to weight ratio of cetyl alcohol to triethyl citrate in said mixture of cetyl alcohol and triethyl citrate may be from 0.25:1 to 0.5:1, preferably from 0.3:1 to 0.45:1, more preferably from 0.35:1 to 0.4:1, and even more preferably from 0.38:1 to 0.4:1 (w/w).

The enteric coating optionally comprises an anti-sticking agent. Suitable anti-sticking agents include dimethicone and castor oil. Dimethicone, in particular dimethicone 1000, is the preferred anti-sticking agent. The anti-sticking agent is usually present in the enteric coating in an amount of between 1.5% and 3% by weight relative to the film-forming agent. Synthetic oils are not to be regarded as preferred anti-sticking agents. The foregoing list of anti-sticking agents is not meant to be exhaustive but merely illustrative, as a person or ordinary skill in the art would understand that many other anti-sticking agents or combination of anti-sticking agents could also be used.

Another embodiment provides a process for the manufacture of pancreatin micropellets, comprising the steps of:
aa. providing pancreatin micropellets cores wherein the pancreatin micropellet cores are substantial free of synthetic oils;
bb. providing an enteric-coating solution comprising
   i. at least one film-forming agent;
   ii. a plasticizer in an amount greater than 1.5 % by weight relative to the at least one film-forming agent; and
   iii. optionally, at least one anti-sticking agent, and
   iv. one or more enzyme-friendly organic solvent(s);
cc. coating the pancreatin micropellet cores with the enteric-coating solution wherein the product temperature of the pancreatin micropellet cores during coating is kept at a temperature suitable for applying the enteric-coating solution; and
dd. drying the coated pancreatin micropellet cores.

In the foregoing process for producing pancreatin micropellets, the film-forming agent(s), the plasticizer(s), the anti-sticking agent(s) and the enzyme-friendly organic solvents generally have the meanings as previously set forth. Preferably, the pancreatin micropellet cores which are provided in process step aa.) and which are substantially free of synthetic oils are produced according to the process for the manufacture of pancreatin micropellet cores as described above.

Due to the process for producing pancreatin micropellets, viz. the coating process as described herein, pharmaceutical acceptable residual amounts of the enzyme-friendly organic solvent(s) present in the enteric-coating solution may still be present in the pancreatin micropellet after drying. It is understood that pancreatin micropellets comprising pharmaceutically acceptable residual amounts of enzyme-friendly organic solvent(s) are within the scope of the present invention.

Process step bb.) may be performed at a temperature between 15 °C and 60 °C. Performing process step bb.) at ambient temperature (i.e. room temperature, approximately between 20 °C and 30 °C), is preferred. Examples of suitable enzyme-friendly organic solvents include acetone, 2-butanol, tert.-butanol, chloroform, dichloromethane, ethanol, methanol, 1-propanol, 2-propanol and mixtures of said solvents. Acetone, ethanol and 2-propanol or their mixtures are preferred as enzyme-friendly organic solvents. Acetone is most preferred. The foregoing list of enzyme-friendly organic solvents in process step bb.) is not meant to be exhaustive but merely illustrative, as a person or ordinary skill in the art would understand that many other enzyme-friendly organic solvents or combination of solvents could also be used.

The enzyme-friendly organic solvent is typically used in an amount between 6 and 10 times, preferably between 7 and 8 times, the weight of the non-solvent coating constituents used to prepare the pancreatin micropellet according to the invention. For example, if the non-solvent coating constituents make up to a total weight of 1.5 g, then 9 g to 15 g of enzyme-friendly organic solvent may be used in process step aa).

In process step cc.) the product temperature of the pancreatin micropellet core, in one embodiment, is usually maintained between 30° C and 60° C while coating, preferably between 32 °C and 55 °C, more preferred between 35 °C and 50 °C, most preferably between 37 °C and 49 °C. Where in process step cc.) cetyl alcohol or a mixture of cetyl alcohol and triethyl citrate is used the product temperature of the pancreatin micropellet core is preferably maintained between 40° C and 46° C (range limits included). Maintaining the product temperature of the pancreatin micropellet cores within the preferred temperature ranges while coating results in improved gastric-acid resistant properties of the pancreatin micropellets, in particular when the enteric coating comprise mixtures of cetyl alcohol and triethyl citrate as plasticizers. The coating in process step cc.) can be accomplished by any process or method known to a person of ordinary skill in the art. Spray coating is preferred. Usually, process step cc.) is performed in a way that the enteric coating comprises between 20% and 30% by weight, preferably between 22% and 26% by weight and more preferably between 22.5 % and 25 % by weight of the total composition of the pancreatin micropellet. The exact parameters to be applied in process step cc.) to achieve the desired enteric coating will depend on the coating technique used. The person skilled in the art understands how to achieve coating films of a desired thickness when using different coating techniques.

Drying of the enteric-coated pancreatin micropellet cores in process step dd.) is usually performed between 30 °C and 75 °C, preferably between 30 °C and 55 °C, more preferably between 35 °C and 50 °C, and for a period of between 6 hours and 60 hours, preferably for a period of between 10 hours and 36 hours.

Pancreatin micropellets according to the invention are particularly suitable for delivery of pancreatin and its digestive enzyme constituents to the upper intestine, in particular to the small intestine, usually to the duodenum, of mammals such as humans. Thus, pancreatin micropellets according to the invention are useful for the prophylaxis and/or treatment of various medical conditions and digestive disorders including pancreatic exocrine insufficiency of different origins like maldigestion, and/or for the prophylaxis and/or treatment of pancreatitis, cystic fibrosis, diabetes type I and/or diabetes type II in mammals such as humans. Maldigestion in mammals such as humans is usually based on a deficiency of digestive enzymes, in particular on a deficiency of endogenous lipase, but also of protease and/or amylase. The cause of such a deficiency of digestive enzymes is frequently a hypofunction of the pancreas (e.g. pancreatic insufficiency, usually known as pancreatic exocrine insufficiency), the organ which produces the largest quantity of, and the most important, endogenous digestive enzymes. If the pancreatic insufficiency is pathological, it may be congenital or acquired. Acquired chronic pancreatic insufficiency may, for example, result from alcoholism. Congenital pancreatic insufficiency may, for example, result from disease such as cystic fibrosis. The consequences of the deficiency of digestive enzymes may be severe symptoms of under-nutrition and malnutrition, which may be accompanied by increased susceptibility to secondary illnesses. In one specific embodiment, pancreatin micropellets according to the invention are therefore particularly suited for treating pancreatic exocrine insufficiency of any origin.

In another embodiment, pancreatin micropellets are provided as previously described, for the manufacture of a medicament for the treatment of medical conditions such as digestive disorders, pancreatic exocrine insufficiency, pancreatitis, cystic fibrosis, diabetes type I and/or diabetes type II.

In yet another embodiment, a method is provided for the treatment of a medical condition such as digestive disorders, pancreatic exocrine insufficiency, pancreatitis, cystic fibrosis, diabetes type I and/or diabetes type II by administering a therapeutically effective amount of pancreatin micropellets previously described to a mammalian subject in need of such treatment.

A further embodiment includes a pharmaceutical composition comprising a pharmacologically effective amount of pancreatin wherein the pancreatin is in the form of pancreatin micropellets manufactured according to the processes described herein in a dosage form suitable for oral administration containing said pharmacologically effective amount of pancreatin.

For proper delivery of an acid-labile drug like pancreatin to the upper intestine of a mammal such as a human, it is necessary that an enteric coating be gastric acid resistant up to a pH of e.g. 5.5. Subsequently, the acid-labile drug will need to be released to the upper intestine which means that the enteric coating must release the acid-labile drug in a less acidic environment, e.g. at pH 5.5 or higher, in particular at a pH of 6. The pancreatin micropellets described herein possess superior gastric acid resisting and protective properties, e.g. superior protective properties at pH 1 and/or pH 5. Pancreatin micro pellets according to the invention wherein the plasticizer is a mixture of cetyl alcohol and triethyl citrate as described above ("CA/TEC-Compositions") are preferred in this regard. Further, CA/TEC-Compositions in general preserve a higher lipase content and usually possess a lower water content relative to other pancreatin micropellets where other plasticizers are used. Furthermore, CA/TEC-Compositions exhibit a favorable dissolution profile which is comparable to the presently marketed pancreatin containing medicaments, e.g. to medicaments known under the trade name Creon^{™}.

In other embodiments of the invention, a pharmaceutical pack or kit is provided comprising one or more containers filled with the pancreatin micropellets described herein. Associated with such container(s) can be various written materials such as instructions for use, or a notice in the form prescribed by a governmental agency regulating the manufacture, use or sale of pharmaceuticals products, which notice reflects approval by the agency of manufacture, use, or sale for human or veterinary administration.

### EXAMPLES

The following examples are meant to be illustrative and not to limit the present disclosure. Other suitable modifications and adaptations are of the variety normally encountered by those skilled in the art and are fully within the spirit and scope of the present invention.

### A. Preparation of pancreatin micropellet cores and pancreatin micropellets

### 1. Preparation of uncoated pancreatin micropellet cores

15.9 kg of pancreatin was mixed with 3.975 kg of polyethylene glycol 4000 in a commercially available high share mixer and thoroughly moistened with 3.975 kg of 2-propanol. The resulting mixture was extruded by means of a commercially available extruding press which was equipped with a piercing die having 0.8 mm internal diameter bores and a cutting device arranged downstream. The temperature was less than 50 °C while pressing. The extruded mass was cut into extrudate fragments of approximately 5 mm length by means of the cutting device.

The resulting 14.64 kg of the extrudate fragments were transferred in four portions of roughly equal size to a commercially available rounding apparatus and rounded off to give approximately elliptically or approximately spherically shaped micropellet cores. An additional 135 g of 2-propanol was added while rounding.

After drying in a commercially available continuous vacuum dryer (Vötsch type) at a temperature in a range from between 35 °C and 50 °C for 12 hours, the pancreatin micropellets were graded, first with a 3.15 mm sieve (sieving of oversize grain > 3.15 mm) and then with a 0.7 mm sieve (sieving of undersize grain < 0.7 mm) and afterwards with a 1.25 mm sieve (sieving of oversize grain > 1.25 mm) to yield 11.98 kg of pancreatin micropellet cores having a pancreatin content of 80 % and a bulk density of 0.67 g/ml.

### 2. Enteric coating of pancreatin micropellet cores

A coating solution was prepared by adding 1623.2 g of hydroxypropyl methylcellulose phthalate (HP 55), 90.2 g of triethyl citrate, 34.3 g of cetyl alcohol and 38.9 g of dimethicone 1000 to 14030 g of acetone at room temperature while stirring.

5025 g of pancreatin micropellet cores (prepared analogously to the process as described herein) were fed into a commercially available fluid bed coater and were spray-coated at a spray rate of 97-101 kg/h and an air pressure of 1.7 bar with the coating solution as prepared above until the desired film-thickness of the coating had been reached. The product temperature of the pancreatin micropellet cores was monitored and maintained in the range between 37 °C and 43 °C during coating. The resulting pancreatin micropellets were then dried in a commercially available vacuum dryer (Vötsch type) at a temperature in a range between 35 °C and 50 °C four 12 hours. The dried pancreatin micropellets were then graded, first with a 0.7 mm sieve (sieving of undersize grain < 0.7 mm) and then with a 1.6 mm sieve (sieving of oversize grain > 1.6 mm) to yield 6532 g of pancreatin micropellets having a pancreatin content of 60 % relative to the enteric-coated pancreatin micropellets. The bulk density of the pancreatin micropellets was 0.69 g/ml.

Further pancreatin micropellets were prepared according to the procedure described above and different coatings were applied in a manner similar to the coating process set forth above to yield additional pancreatin micropellets. The compositions of the additional pancreatin micro pellets and certain process parameters from the coating processes are given below in Table 1. Composition G can be produced according to processes as described in U.S. Pat. No. 5,378,462. Comparative composition H was prepared according to a process as described above which has been slightly modified (i.e. dibutylphthalate was used as a plasticizer in the coating). All batches have been produced in laboratory scale except where otherwise indicated.

**Table 1: Composition of (enteric-coated) pancreatin micropellets and applicable process parameters**

| | | **Composition** | | | | | |
|---|---|---|---|---|---|---|---|
| **Ingredients mg/capsule** | | **A** | **B** | **C** | **D** | **1** | **2** |
| **Micropellet Cores** | Pancreatin | 150.00 | 150.00 | 150.00 | 150.00 | 150.00 | 150.00 |
| | PEG 4000 | 37.50 | 37.50 | 37.50 | 37.50 | 37.50 | 37.50 |
| **Enteric Coating (film)** | HP 55 | 48.60 | 48.60 | 48.60 | 48.60 | 48.60 | 48.60 |
| | Dimethicone | 1.25 | 1.25 | 1.25 | 1.25 | 1.25 | 1.25 |
| | TEC | 0 | 0 | 3.0 | 4.10 | 5.00 | 0 |
| | CA | 0 | 0.40 | 0 | 0 | 0 | 1.00 |
| | Sum | 237.40 | 237.75 | 240.35 | 241.45 | 242.4 | 238.35 |
| **Process parameters** | Pellet temp. while coating | 40°C | 40°C | 40°C | 40°C | 40°C | 40°C |

| | | **Composition** | | | | | |
|---|---|---|---|---|---|---|---|
| **Ingredients mg/capsule** | | **3** | **4** | **5** | **6*** | **7** | **8** |
| **Micropellet Cores** | Pancreatin | 150.00 | 150.00 | 150.00 | 150.00 | 150.00 | 150.00 |
| | PEG 4000 | 37.50 | 37.50 | 37.50 | 37.50 | 37.50 | 37.50 |
| **Enteric Coating (film)** | HP 55 | 52.60 | 48.60 | 48.60 | 52.25 | 52.25 | 52.25 |
| | Dimethicone | 1.25 | 1.25 | 1.25 | 1.25 | 1.25 | 1.25 |
| | TEC | 0 | 3.60 | 3.00 | 2.90 | 2.90 | 2.90 |
| | CA | 1.15 | 0.40 | 1.00 | 1.10 | 1.10 | 1.10 |
| | Sum | 242.50 | 241.35 | 241.35 | 245.00 | 245.00 | 245.00 |
| **Process parameters** | Pellet temp. while coating | 40°C | 40°C | 40°C | 40°C | 30°C | 35°C |

| | | **Composition** | | | | | |
|---|---|---|---|---|---|---|---|
| **Ingredients mg/capsule** | | **9** | **10** | **11** | **12** | **13** | **14** |
| **Micropellet Cores** | Pancreatin | 150.00 | 150.00 | 150.00 | 150.00 | 150.00 | 150.00 |
| | PEG 4000 | 37.50 | 37.50 | 37.50 | 37.50 | 37.50 | 37.50 |
| **Enteric Coating (film)** | HP 55 | 56.34 | 56.34 | 56.34 | 52.25 | 52.25 | 56.34 |
| | Dimethicone | 1.35 | 1.35 | 1.35 | 1.25 | 1.25 | 1.35 |
| | TEC | 3.13 | 3.13 | 3.13 | 2.90 | 2.90 | 3.13 |
| | CA | 1.19 | 1.19 | 1.19 | 1.10 | 1.10 | 1.19 |
| | Sum | 249.51 | 249.51 | 249.51 | 245.00 | 245.00 | 249.51 |
| **Process parameters** | Pellet temp. while coating | 37 °C | 40 °C | 43 °C | 49 °C | 40 °C | 46 °C |

| **Ingredients mg/capsule** | | **15** | **E** | **F** | **G** | **H** | |
|---|---|---|---|---|---|---|---|
| **Micropellet Cores** | Pancreatin | 128.06 | 150.00 | 150.00 | 150.00 | 150.00 | |
| | PEG 4000 | 32.01 | 37.50 | 37.50 | 37.50 | 37.50 | |
| | Light mineral oil | 0 | 0 | 0 | 3.75 | 0 | |
| **Enteric Coating (film)** | HP 55 | 48.10 | 48.60 | 48.60 | 48.60 | 48.60 | |
| | Dimethicone | 1.15 | 1.25 | 1.25 | 1.25 | 1.25 | |
| | TEC | 2.67 | 1.00 | 2.00 | 0 | 0 | |
| | CA | 1.01 | 0 | 0 | 0 | 0 | |
| | DBP | 0 | 0 | 0 | 4.10 | 4.10 | |
| | Light mineral oil | 0 | 0 | 0 | 3.30 | 0 | |
| | Sum | 213.00 | 238.35 | 239.35 | 248.50 | 241.50 | |
| **Process parameters** | Pellet temp. while coating | n.a. | 40 °C | 40°C | 40°C | 40 °C | |

| | | | | | | | |
|---|---|---|---|---|---|---|---|
| PEG=polyethylene glycol; TEC=triethyl citrate; CA=cetyl alcohol; HP 55=hydroxypropyl methylcellulose phthalate; temp.=temperature; DBP=dibutyl phthalate; *=production scale; n.a.:data not available. Composition G is a currency available high-quality pharmaceutical composition comprising pancreatin and light mineral oil. Compositions No. 6, 10, 13, 14 and 15 are examples of preferred CA/TEC compositions. Composition No. 3 is an example of a preferred composition comprising cetyl alcohol as the sole plasticizer. | | | | | | | |

### B. Determination of the gastric acid resistance of enteric-coated pancreatin micropellets at pH 1 and pH 5

The gastric acid resistances of the pancreatin micropellet s (see Table 1 here above) were measured.

Resistance to gastric juice (pH 1) of the different pancreatin micropellets from Table 1 was determined by immersing the pancrelipase micropellets for 2 hours in 0.1 mol/l hydrochloric acid in a disintegration tester according to the European Pharmacopoeia (Ph. Eur.). Then the un-dissolved portion of the pellets was separated from the solution and their residual lipase activity was determined according to the lipase assay of Ph. Eur./ The international Pharmaceutical Federation" (FIP), PO Box 84200; 2508 AE The Hague; The Netherlands. The results of these tests for gastric resistance of the enteric coating are presented in Table 2 ("stability at pH1").

Further, a similar test at pH 5 was performed using the same conditions as outlined in the previous paragraph, with the exception that a phosphate buffer pH 5.0 (2.0 g sodium chloride and 9.2 g sodium di-hydrogen phosphate monohydrate per liter adjusted to pH 5.0) was used as a solvent instead of 0.1 mol/l hydrochloric acid. The results of these tests for gastric resistance are also presented below in Table 2 ("stability at pH5").

The gastric acid resistances of the pancreatin micro pellets from Table 1 (see above) are each given in Table 2 as percentages of the residual lipolytic activity after the incubation in relation to the actual lipolytic activity of the samples tested prior to the incubation (relative gastric acid resistance). The lipolytic activity is determined according to the lipase assay described in the United States Pharmacopoeia (USP) monograph "pancrelipase delayed-release capsules". In principle, any standardized and characterized pancreatin sample may be used as the lipase reference standard. For example, a predetermined lipolytic activity standard may be obtained from the "International Pharmaceutical Federation" (FIP), PO Box 84200; 2508 AE The Hague; The Netherlands. For the purposes of the present invention, an internal pancreatin standard was used which is available on request from Solvay Pharmaceuticals GmbH, Hans-Boeckler-Allee 20, 30173 Hannover, Germany.

**Table 2: Relative gastric acid resistances (stabilities) of the pancreatin micropellets at pH1 and pH5**

| **Composition** | **Stability at pH 5 [%]** | **Stability at pH 1 [%]** |
|---|---|---|
| **A** | 15.3 | 15.9 |
| **B** | 63.2 | 53.8 |
| **C** | 71.6 | 84.2 |
| **D** | 52.0 | 93.6 |
| **1** | 87.0 | 96.0 |
| **2** | 76.4 | 92.6 |
| **3** | 92.1 | 94.5 |
| **4** | 85.3 | 93.7 |
| **5** | 92.0 | 93.0 |
| **6** | 94.9 | 99.4 |
| **7** | 67.4 | 89.8 |
| **8** | 80.5 | 95.2 |
| **9** | 83.8 | 90.8 |
| **10** | 97.9 | 99.6 |
| **11** | 89.0 | 93.5 |
| **12** | 83.7 | 94.8 |
| **13** | 100.2 | 102.7 |
| **14** | 93.6 | 98.7 |
| **E** | 48.6 | 65.0 |
| **F** | 36.5 | 75.0 |
| **G** | 98.6 | 100.6 |

Preferred pancreatin micropellets have a gastric acid resistance (stability) at pH 1 of at least 75 %, in particular of at least 85 %, preferably of at least 90 %, more preferred of at least 95 %, relative to a predetermined pancreatin lipolytic activity standard.

Other preferred pancreatin micropellets as disclosed herein have a gastric acid resistance at pH 5 of at least 75 %, in particular of at least 85 %, preferably of at least 90 %, more preferred of at least 95 %, relative to a predetermined pancreatin lipolytic activity standard.

Pancreatin micro pellets which are most preferred have a gastric acid resistance at pH 1 of at least 90 % and an additional gastric acid resistance at pH 5 of at least 90 %, relative to a predetermined pancreatin lipolytic activity standard.

### C. Determination of the dissolution profile of enteric coated pancreatin micropellets

The dissolution profile of different enteric coated pancreatin micro pellets from Table 1 (see above) was determined according to a test procedure as described in the United States Pharmacopoeia (USP) monograph "pancrelipase delayed-release capsules" with increased gastric resistance phase.

The determination of the resistance to gastric fluid was performed using gastric juice without enzymes according to USP under standardized conditions (37°C, 100 rpm) for 2 hours in the dissolution apparatus (basket apparatus USP). Then the un-dissolved portion of the enteric coated pancreatin micropellets was separated from the solution and transferred into the paddle apparatus according to USP, filled with phosphate buffer solution at pH 6.0 to determine the dissolution of enzymes. The enteric coated pancreatin micropellets were agitated in a dissolution tester under standardized conditions for usually 90 minutes (see exact timepoints in Table 3 below) at 37 °C and 50 rpm.

The lipase activity was determined after selected time points (see Table 3) according to the lipase assay described in the USP monograph "pancrelipase delayed-release capsule".

The results of the dissolution profile test are presented as "% residual lipase activity of actual lipase activity" below (see Table 3).

**Table 3: Dissolution profiles of the enteric coated pancreatin micropellets in phosphate buffer (n.a.: data not available)**

| **Time points [min.]** | **% lipase activity of initial actual activity for pancreatin micropellet composition No.** | | |
|---|---|---|---|
| | **G** | **H** | **14** |
| **5** | 0.0 | 4 | n.a. |
| **10** | 0.0 | 6.25 | 15,37 |
| **15** | 11.9 | 23.15 | 34,38 |
| **20** | 48.0 | 48.15 | n.a. |
| **25** | 62.3 | 62.9 | n.a. |
| **30** | 73.5 | 69.6 | 73,86 |
| **45** | 77.1 | 77.15 | 84,45 |
| **60** | 79.9 | 78.35 | 81,25 |
| **75** | 78.4 | 76.7 | 80,40 |
| **90** | 78.2 | 75.25 | .n.a. |

For the dissolution profile test results as provided in Table 3, a comparison of the compositions "G" and "H" was performed. Said comparison was based on the "Guidance for Industry", SUPAC-MR, Modified Release Solid Oral Dosage Forms (September 1997) by calculating the similarity factor (f2)**.** The 2 acceptance limits for determining similarity of two compared curves were (i) a factor (f2) > 50 and (ii) the average deviation at any dissolution sampling point should not be greater than 15 %.

When applying the above-stated acceptance limits for determining similarity (f2 = 71.8) it was found that the dissolution profile of pancreatin micropellet composition "H" (see Table 1) could be considered to be similar to the dissolution profile of the reference pancreatin micropellet composition "G" (see Table 1).

Where in the present disclosure numeric values are given as ranges, the respective range limits are generally meant to be included in and being part of the given ranges unless expressly stated otherwise.

The use of the terms "a" and "an" and "the" and similar references in the context of this disclosure (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., such as, preferred, preferably) provided herein, is intended merely to further illustrate the content of the disclosure and does not pose a limitation on the scope of the claims. No language in the specification should be construed as indicating any non-claimed element as essential to the practice of the invention.

Accordingly, this invention includes all modifications and equivalents of the subject matter recited in the claims appended hereto as permitted by applicable law. Moreover, any combination of the above described elements in all possible variations thereof is encompassed by the invention unless otherwise indicated herein or otherwise clearly contradicted by context.

## Claims

1. A process for the manufacture of pancreatin micropellet cores, comprising the steps of:
a. preparing an extrudable mixture comprising:
i. 10% to 95% pancreatin;
ii. 5% to 90% of at least one pharmaceutically acceptable binding agent;,
iii. 0% to 10% of at least one pharmaceutically acceptable excipient; and
iv. one or more enzyme-friendly organic solvents in an amount sufficient to form an extrudable mixture;
wherein the percentages of components are weight to weight of the pancreatin micropellet cores and the constituents i.), ii.) and iii.) add to 100 % by weight;
b. extruding the extrudable mixture to create pancreatin micropellet cores ;
c. forming the pancreatin micropellet cores into approximately spherical or approximately ellipsoidal shape in the presence of additional enzyme-friendly organic solvent; and
d. removing the one or more enzyme-friendly organic solvents from the pancreatin micropellet cores such that the pancreatin micropellet cores are substantially free of the one or more enzyme-friendly organic solvents;
wherein the pancreatin micropellet cores are substantially free of synthetic oils.

2. The process of Claim 1 wherein the pancreatin is present from 70% to 90% weight to weight and the binding agent is present from 10% to 30% weight to weight of the pancreatin micropellet cores.

3. The process of Claim 1 wherein no pharmaceutically acceptable excipient is present.

4. The process of Claim 1 wherein the binding agent is selected from the group consisting of: polyethylene glycol 1500, polyethylene glycol 2000, polyethylene glycol 3000, polyethylene glycol 4000, polyethylene glycol 6000, polyethylene glycol 8000, polyethylene glycol 10000, hydroxypropyl methylcellulose, polyoxyethylen, copolymers of polyoxyethylen-polyoxypropylen and mixtures of said organic polymers.

5. The process of Claim 1 wherein the binding agent is polyethylene glycol 4000.

6. A pancreatin micropellet core, obtainable by the process of Claim 1.

7. A pancreatin micropellet comprising a pancreatin micropellet core as defined in Claim 6 and an enteric coating.

8. A pancreatin micropellet as defined in Claim 7, wherein the enteric coating does not comprise synthetic oils.

9. A pancreatin micropellet as defined in Claim 8, wherein the enteric coating comprises
aa. at least one film-forming agent selected from the group consisting of: agar, carbomer polymers, carboxymethyl cellulose, carboxymethyl ethyl cellulose, carrageen, cellulose acetate phthalate, cellulose acetate succinate, cellulose acetate trimelliate, chitin, corn protein extract, ethyl cellulose, gum arabic, hydroxypropyl cellulose, hydroxypropylmethyl acetate succinate, hydroxypropyl methylcellulose acetate succinate, hydroxypropyl methylcellulose phthalate, methacrylic acid-ethyl methacrylate-copolymer, methyl cellulose, pectin, polyvinyl acetate phthalate, polivinyl alcohol, shellac, sodium alginate, starch acetate phthalate, styrene/maleic acid copolymer and mixtures of said film-forming polymers;
bb. a plasticizer selected from the group consisting of triethyl citrate, cetyl alcohol and mixtures of triethyl citrate and cetyl alcohol, in an amount greater than 1.5 % by weight relative to the at least onefilm-forming agent; and
cc. optionally, at least one anti-sticking agent.

10. The pancreatin micropellet of Claim 9 wherein the film-forming agent is hydroxypropyl methylcellulose phthalate.

11. The pancreatin micropellet of Claim 9 wherein the plasticizer is comprised of cetyl alcohol and triethyl citrate which are collectively present in an amount greater than 3% by weight relative to the film-forming agent.

12. The pancreatin micropellet of Claim 9 wherein the plasticizer is cetyl alcohol present in an amount greater than 1.5% by weight relative to the film-forming agent.

13. A process for the manufacture of pancreatin micropellets, comprising the steps of:
aa. providing pancreatin micropellet cores as defined in Claim 6;
bb. providing an enteric-coating solution comprising
i. at least one film-forming agent selected from the group consisting of: agar, carbomer polymers, carboxymethyl cellulose, carboxymethylethyl cellulose, carrageen, cellulose acetate phthalate, cellulose acetate succinate, cellulose acetate trimelliate, chitin, corn protein extract, ethyl cellulose, gum arabic, hydroxypropyl cellulose, hydroxypropylmethyl acetate succinate, hydroxypropyl methylcellulose acetate succinate, hydroxypropyl methylcellulose phthalate, methacrylic acid-ethyl methacrylate-copolymer, methyl cellulose, pectin, polyvinyl acetate phthalate, polivinyl alcohol, shellac, sodium alginate, starch acetate phthalate, styrene/maleic acid copolymer and mixtures of said film-forming polymers;
ii. a plasticizer selected from the group consisting of triethyl citrate, cetyl alcohol and mixtures of triethyl citrate and cetyl alcohol, in an amount greater than 1.5 % by weight relative to the at least one film-forming agent; and
iii. optionally, at least one anti-sticking agent, and
iv. one or more enzyme-friendly organic solvent(s);
cc. coating the pancreatin micropellet cores with the enteric-coating solution wherein the product temperature of the pancreatin micropellet cores during coating is kept at a temperature suitable for applying the enteric-coating solution; and
dd. drying the coated pancreatin micropellet cores.

14. A pharmaceutical composition comprising a pharmacologically effective amount of pancreatin micropellet cores as defined in Claim 6 or pancreatin micropellets as defined in Claim 7.

15. The pharmaceutical composition of Claim 14 wherein the pancreatin micropellet cores or the pancreatin micropellets are in a dosage form suitable for oral administration.

16. The pharmaceutical composition of Claim 14 or Claim 15 wherein the pancreatin micropellet cores, the pancreatin micropellets and/or their respective oral dosage forms are further incorporated in at least one outer package selected from capsules, sachets, blisters or bottles.

17. Pancreatin micropellet cores as defined in Claim 6 or pancreatin micropellets as defined in Claim 7 for the use in the treatment of digestive disorders, pancreatic exocrine insufficiency, pancreatitis, cystic fibrosis, diabetes type I and/or diabetes type II.

## Patentansprüche

1. Verfahren zur Herstellung von Pankreatinmikropelletkernen, bei dem man:
a. eine extrudierbare Mischung, enthaltend:
i. 10% bis 95% Pankreatin;
ii. 5% bis 90% mindestens eines pharmazeutisch annehmbaren Bindemittels;
iii. 0% bis 10% mindestens eines pharmazeutisch annehmbaren Hilfsstoffs und
iv. ein oder mehrere enzymfreundliche organische Lösungsmittel in einer zur Bildung einer extrudierbaren Mischung ausreichenden Menge
herstellt; wobei sich die Prozentanteile der Komponenten auf das Gewicht der Pankreatinmikropelletkerne beziehen und sich die Bestandteile i.), ii.), und iii.) zu 100 Gew.-% summieren;
b. die extrudierbare Mischung zur Bildung von Pankreatinmikropelletkernen extrudiert;
c. die Pankreatinmikropelletkerne in Gegenwart von zusätzlichem enzymfreundlichem organischem Lösungsmittel in ungefähr sphärische oder ungefähr elliposide Form bringt und
d. das oder die enzymfreundlichen organischen Lösungsmittel aus den Pankreatinmikropelletkernen entfernt, so daß die Pankreatinmikropelletkerne weitgehend frei von dem oder den enzymfreundlichen organischen Lösungsmitteln sind;
wobei die Pankreatinmikropelletkerne weitgehend frei von synthetischen Ölen sind.

2. Verfahren nach Anspruch 1, bei dem das Pankreatin in einer Menge von 70 bis 90 Gew.-% und das Bindemittel in einer Menge von 10 bis 30 Gew.-%, bezogen auf die Pankreatinmikropelletkerne, vorliegt.

3. Verfahren nach Anspruch 1, bei dem kein pharmazeutisch annehmbarer Hilfsstoff vorliegt.

4. Verfahren nach Anspruch 1, bei dem man das Bindemittel aus der Gruppe bestehend aus Polyethylenglykol 1500, Polyethylenglykol 2000, Polyethylenglykol 3000, Polyethylenglykol 4000, Polyethylenglykol 6000, Polyethylenglykol 8000, Polyethylenglykol 10000, Hydroxypropylmethylcellulose, Polyoxyethylen, Polyoxyethylen-Polyoxypropylen-Copolymeren und Mischungen dieser organischen Polymere auswählt.

5. Verfahren nach Anspruch 1, bei dem es sich bei dem Bindemittel um Polyethylenglykol 4000 handelt.

6. Pankreatinmikropelletkern, der nach dem Verfahren gemäß Anspruch 1 erhältlich ist.

7. Pankreatinmikropellet, enthaltend einen Pankreatinmikropelletkern gemäß Anspruch 6 und einen magensaftresistenten Überzug.

8. Pankreatinmikropellet nach Anspruch 7, worin der magensaftresistente Überzug keine synthetischen Öle enthält.

9. Pankreatinmikropellet nach Anspruch 8, worin der magensaftresistente Überzug
aa. mindestens einen Filmbildner aus der Gruppe bestehend aus Agar, Carbomer-Polymeren, Carboxymethylcellulose, Carboxymethylethylcellulose, Carrageen, Celluloseacetatphthalat, Celluloseacetatsuccinat, Celluloseacetattrimellitat, Chitin, Maisproteinextrakt, Ethylcellulose, Gummi arabicum, Hydroxypropylcellulose, Hydroxypropylmethylacetatsuccinat, Hydroxypropylmethylcelluloseacetatsuccinat, Hydroxypropylmethylcellulosephthalat, Methacrylsäure-Ethylmethacrylat-Copolymer, Methylcellulose, Pektin, Polyvinylacetatphthalat, Polyvinylalkohol, Schellack, Natriumalginat, Stärkeacetatphthalat, Styrol/Maleinsäure-Copolymer und Mischungen dieser filmbildenden Polymere;
bb. einen Weichmacher aus der Gruppe bestehend aus Triethylcitrat, Cetylalkohol und Mischungen von Triethylcitrat und Cetylalkohol in einer Menge von mehr als 1,5 Gew.-%, bezogen auf den mindestens einen Filmbildner; und
cc. gegebenenfalls mindestens ein Antihaftmittel enthält.

10. Pankreatinmikropellet nach Anspruch 9, worin es sich bei dem Filmbildner um Hydroxypropylmethylcellulosephthalat handelt.

11. Pankreatinmikropellet nach Anspruch 9, worin der Weichmacher aus Cetylalkohol und Triethylcitrat, die gemeinsam in einer Menge von mehr als 3 Gew.-%, bezogen auf den Filmbildner vorliegen, besteht.

12. Pankreatinmikropellet nach Anspruch 9, worin es sich bei dem Weichmacher um Cetylalkohol, der in einer Menge von mehr als 1,5 Gew.-%, bezogen auf den Filmbildner, vorliegt, handelt.

13. Verfahren zur Herstellung von Pankreatinmikropellets, bei dem man:
aa. Pankreatinmikropelletkerne gemäß Anspruch 6 bereitstellt;
bb. eine Lösung für einen magensaftresistenten Überzug bereitstellt, die
i. mindestens einen Filmbildner aus der Gruppe bestehend aus Agar, Carbomer-Polymeren, Carboxymethylcellulose, Carboxymethylethylcellulose, Carrageen, Celluloseacetatphthalat, Celluloseacetatsuccinat, Celluloseacetattrimellitat, Chitin, Maisproteinextrakt, Ethylcellulose, Gummi arabicum, Hydroxypropylcellulose, Hydroxypropylmethylacetatsuccinat, Hydroxypropylmethylcelluloseacetatsuccinat, Hydroxypropylmethylcellulosephthalat, Methacrylsäure-Ethylmethacrylat-Copolymer, Methylcellulose, Pektin, Polyvinylacetatphthalat, Polyvinylalkohol, Schellack, Natriumalginat, Stärkeacetatphthalat, Styrol/Maleinsäure-Copolymer und Mischungen dieser filmbildenden Polymere;
ii. einen Weichmacher aus der Gruppe bestehend aus Triethylcitrat, Cetylalkohol und Mischungen von Triethylcitrat und Cetylalkohol in einer Menge von mehr als 1,5 Gew.-%, bezogen auf den mindestens einen Filmbildner; und
iii. gegebenenfalls mindestens ein Antihaftmittel und
iv. ein oder mehrere enyzmfreundliche organische Lösungsmittel
enthält;
cc. die Pankreatinmikropelletkerne mit der Lösung für einen magensaftresistenten Überzug beschichtete, wobei man die Produkttemperatur der Pankreatinmikropelletkerne während der Beschichtung bei einer für das Aufbringen der Lösung für einen magensaftresistenten Überzug geeigneten Temperatur hält; und
dd. die beschichteten Pankreatinmikropelletkerne trocknet.

14. Pharmazeutische Zusammensetzung, enthaltend eine pharmakologisch wirksame Menge an Pankreatinmikropelletkernen gemäß Anspruch 6 oder Pankreatinmikropellets gemäß Anspruch 7.

15. Pharmazeutische Zusammensetzung nach Anspruch 14, worin die Pankreatinmikropelletkerne oder Pankreatinmikropellets in einer für die orale Verabreichung geeigneten Dosierungsform vorliegen.

16. Pharmazeutische Zusammensetzung nach Anspruch 14 oder Anspruch 15, worin die Pankreatinmikropelletkerne, die Pankreatinmikropellets und/oder ihre jeweiligen oralen Dosierungsformen ferner in mindestens eine unter Kapseln, Beuteln, Blistern oder Flaschen ausgewählte äußere Verpackung eingearbeitet sind.

17. Pankreatinmikropelletkerne gemäß Anspruch 6 oder Pankreatinmikropellets gemäß Anspruch 7 zur Verwendung bei der Behandlung von Verdauungsstörungen, exokriner Pankreasinsuffizienz, Pankreatitis, zystischer Fibrose, Diabetes Typ I und/oder Diabetes Typ II.

## Revendications

1. Procédé de fabrication de coeurs de micropellets de pancréatine, comprenant les étapes consistant à :
a. préparer un mélange extrudable comprenant :
i. de 10 % à 95 % de pancréatine ;
ii. de 5 % à 90 % d'au moins un agent liant pharmaceutiquement acceptable ;
iii. de 0 % à 10 % d'au moins un excipient pharmaceutiquement acceptable ; et
iv. un ou plusieurs solvants organiques préservant les enzymes, dans une quantité suffisante pour former un mélange extrudable ;
les pourcentages de composants étant en poids sur le poids des coeurs de micropellets de pancréatine et les constituants i.), ii.) et iii.) s'ajoutant à 100 % en poids ;
b. extruder le mélange extrudable pour créer des coeurs de micropellets de pancréatine ;
c. mettre les coeurs de micropellets de pancréatine sous une forme approximativement sphérique ou approximativement ellipsoïdale en présence de plus de solvant organique préservant les enzymes ; et
d. éliminer un ou plusieurs des solvants organiques préservant les enzymes des coeurs de micropellets de pancréatine de sorte que les coeurs de micropellets de pancréatine soient sensiblement dépourvus d'un ou plusieurs des solvants organiques préservant les enzymes ;
les coeurs de micropellets de pancréatine étant sensiblement dépourvus d'huiles de synthèse.

2. Procédé selon la revendication 1, **caractérisé en ce que** la pancréatine est présente dans une quantité de 70 % à 90 % en poids sur le poids et l'agent liant est présent dans une quantité de 10 % à 30 % en poids sur le poids des coeurs de micropellets de pancréatine.

3. Procédé selon la revendication 1, **caractérisé en ce qu'**aucun excipient pharmaceutiquement acceptable n'est présent.

4. Procédé selon la revendication 1, **caractérisé en ce que** l'agent liant est choisi dans le groupe constitué de : polyéthylèneglycol 1500, polyéthylèneglycol 2000, polyéthylèneglycol 3000, polyéthylèneglycol 4000, polyéthylèneglycol 6000, polyéthylèneglycol 8000, polyéthylèneglycol 10000, hydroxypropylméthylcellulose, polyoxyéthylène, copolymères de polyoxyéthylène-polyoxypropylène et des mélanges desdits polymères organiques.

5. Procédé selon la revendication 1, **caractérisé en ce que** l'agent liant est le polyéthylèneglycol 4000.

6. Coeur de micropellet de pancréatine, susceptible d'être obtenu par le procédé de la revendication 1.

7. Micropellet de pancréatine comprenant un coeur de micropellet de pancréatine tel que défini dans la revendication 6 et un enrobage entérique.

8. Micropellet de pancréatine tel que défini dans la revendication 7, **caractérisé en ce que** l'enrobage entérique ne comprend pas d'huiles de synthèse.

9. Micropellet de pancréatine tel que défini dans la revendication 8, **caractérisé en ce que** l'enrobage entérique comprend
aa. au moins un agent filmogène choisi dans le groupe constitué de : agar-agar, polymères de carbomer, carboxyméthylcellulose, carboxyméthyléthylcellulose, carragaheen, acétate phtalate de cellulose, acétate succinate de cellulose, acétate trimelliate de cellulose, chitine, extrait de protéine de maïs, éthylcellulose, gomme arabique, hydroxypropylcellulose, acétate succinate d'hydroxypropylméthyle, acétate succinate d'hydroxypropylméthylcellulose, phtalate d'hydroxypropylméthylcellulose, copolymère acide méthacrylique-méthacrylate d'éthyle, méthylcellulose, pectine, acétate phtalate de polyvinyle, alcool polyvinylique, shellac, alginate de sodium, acétate phtalate d'amidon, copolymère styrène/acide maléique et des mélanges desdits polymères filmogènes ;
bb. un plastifiant choisi dans le groupe constitué de citrate de triéthyle, d'alcool cétylique et des mélanges de citrate de triéthyle et d'alcool cétylique, dans une quantité supérieure à 1,5 % en poids par rapport audit au moins un agent filmogène ; et
cc. éventuellement au moins un agent antiadhérent.

10. Micropellet de pancréatine selon la revendication 9, **caractérisé en ce que** l'agent filmogène est le phtalate d'hydroxypropylméthylcellulose.

11. Micropellet de pancréatine selon la revendication 9, **caractérisé en ce que** le plastifiant est constitué d'alcool cétylique et de citrate de triéthyle qui sont présents conjointement dans une quantité supérieure à 3 % en poids par rapport à l'agent filmogène.

12. Micropellet de pancréatine selon la revendication 9, **caractérisé en ce que** le plastifiant est l'alcool cétylique présent dans une quantité supérieure à 1,5 % en poids par rapport à l'agent filmogène.

13. Procédé de fabrication de micropellets de pancréatine, comprenant les étapes consistant à :
aa. produire des coeurs de micropellets de pancréatine tels que définis dans la revendication 6 ;
bb. produire une solution d'enrobage entérique comprenant
i. au moins un agent filmogène choisi dans le groupe constitué de : agar-agar, polymères de carbomer, carboxyméthylcellulose, carboxyméthyléthylcellulose, carragaheen, acétate phtalate de cellulose, acétate succinate de cellulose, acétate trimelliate de cellulose, chitine, extrait de protéine de maïs, éthylcellulose, gomme arabique, hydroxypropylcellulose, acétate succinate d'hydroxypropylméthyle, acétate succinate d'hydroxypropylméthylcellulose, phtalate d'hydroxypropylméthylcellulose, copolymère acide méthacrylique-méthacrylate d'éthyle, méthylcellulose, pectine, acétate phtalate de polyvinyle, alcool polyvinylique, shellac, alginate de sodium, acétate phtalate d'amidon, copolymère styrène/acide maléique et des mélanges desdits polymères filmogènes ;
ii. un plastifiant choisi dans le groupe constitué de citrate de triéthyle, d'alcool cétylique et des mélanges de citrate de triéthyle et d'alcool cétylique, dans une quantité supérieure à 1,5 % en poids par rapport à au moins un agent filmogène ; et
iii. éventuellement au moins un agent antiadhérent, et
iv. un ou plusieurs solvants organiques préservant les enzymes ;
cc. enrober les coeurs de micropellets de pancréatine avec une solution d'enrobage entérique, la température du produit des coeurs de micropellets de pancréatine pendant l'enrobage étant maintenue à une température convenable pour appliquer la solution d'enrobage entérique ; et
dd. sécher les coeurs de micropellets de pancréatine enrobés.

14. Composition pharmaceutique comprenant une quantité pharmacologiquement efficace de coeurs de micropellets de pancréatine tels que définis dans la revendication 6 ou de micropellets de pancréatine tels que définis dans la revendication 7.

15. Composition pharmaceutique selon la revendication 14, **caractérisée en ce que** les coeurs de micropellets de pancréatine ou les micropellets de pancréatine sont sous une forme de dosage convenable pour l'administration par voie orale.

16. Composition pharmaceutique selon la revendication 14 ou la revendication 15, **caractérisée en ce que** les coeurs de micropellets de pancréatine, les micropellets de pancréatine et/ou leurs formes de dosage orales respectives sont en outre incorporés dans au moins un conditionnement externe choisi parmi les capsules, les sachets, les plaquettes ou les bouteilles.

17. Coeurs de micropellets de pancréatine tels que définis dans la revendication 6 ou micropellets de pancréatine tels que définis dans la revendication 7, destinés à être utilisés dans le traitement des troubles digestifs, de l'insuffisance exocrine pancréatique, de la pancréatite, de la mucoviscidose, du diabète de type I et/ou du diabète de type II.
